(19) Europäisches Patentamt
European Patent Office
Office européen des brevets

(11) Publication number: **0 129 163**
**B1**

(12) # EUROPEAN PATENT SPECIFICATION

(45) Date of publication of patent specification: **16.09.87**

(21) Application number: **84106562.6**

(22) Date of filing: **08.06.84**

(51) Int. Cl.$^4$: **C 07 C 101/34,** C 07 C 99/00, C 07 C 129/12, C 07 C 149/243, C 07 D 207/08, A 61 K 31/195

(54) **Arphamenine and its related compounds, a process for their preparation and their use as medicaments.**

(30) Priority: **17.06.83 JP 107888/83**

(43) Date of publication of application:
**27.12.84 Bulletin 84/52**

(45) Publication of the grant of the patent:
**16.09.87 Bulletin 87/38**

(84) Designated Contracting States:
**AT BE CH DE FR GB IT LI LU NL SE**

(56) References cited:
**US-A-3 843 696**

**JOURNAL OF MEDICINAL CHEMISTRY, vol. 24, July 1981 R.F. MEYER et al. "Novel Synthesis of (S)-1-(5-(Benzoyl-amino)-1,4-dioxo-6-phenylhexyl)-L-proline and Analogues: Potent Angiotensin Converting Enzyme Inhibitors" pages 964-969**

**TETRAHEDRON LETTERS, vol. 23, no. 25, 1982, Oxford, New York, Paris, Frankfurt C. JENNINGS-WHITE et al. "Synthesis of Ketomethylene analogs of Di-peptides" pages 2533, 2534**

(73) Proprietor: **MICROBIAL CHEMISTRY RESEARCH FOUNDATION**
**14-23, Kamiosaki 3 Chome Shinagawa-ku**
**Tokyo 141 (JP)**

(72) Inventor: **Umezawa, Hamao, Prof.**
**4-23, Toyotama-kita**
**Nerima-ku Tokyo (JP)**
Inventor: **Aoyagi, Takaaki**
**3-3-6, Honkugenuma**
**Fujisawa-city Kanagawa Prefecture (JP)**
Inventor: **Tatsuta, Kuniaki**
**2-26-7, Matsunoki**
**Suginami-ku Tokyo (JP)**
Inventor: **Fukatsu, Shunzo**
**1-13, Ichigaya-tamachi**
**Shinjuku-ku Tokyo (JP)**
Inventor: **Nakamura, Takeshi**
**4-604, Kodan Daigo Ishida-Danchi Ishidasakuragi 3**
**Fushimi-ku Kyoto City Kyoto (JP)**

(74) Representative: **Meyer-Dulheuer, Karl-Hermann, Dr. et al**
**HOECHST Aktiengesellschaft Zentrale**
**Patentabteilung Postfach 80 03 20**
**D-6230 Frankfurt/Main 80 (DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

EP 0 129 163 B1

Courier Press, Leamington Spa, England.

**Description**

The present invention relates to methods of synthesizing Arphamenine and its related compounds, expressed by the following general formula (I)

$$R{-}CO{-}CH_2{-}\underset{*}{\overset{R_2}{\overset{|}{C}}}H{-}COOH \qquad (I)$$

wherein R represents an α-amino acid residue corresponding to an α-amino acid molecule from which the α-carboxyl group has been removed, $R^2$ represents a hydrogen atom, an alkyl group having 1 to 6 carbon atoms, an aryl or aralkyl group, both of which may be unsubstituted or substituted in the nucleus and * represents a configuration of the R- or S-type or the R- and S-types combined.

Arphamenines A and B of the general formula (I) in which R represents an α-amino acid residue derived from arginine and $R^2$ represents a benzyl group or a p-hydroxybenzyl group — referred to generally as Arphamenine — are novel substances that were discovered by the present inventors as aminopeptidase-inhibitory substances on the basis of screening. They are produced by fermentation, and they also have an immunopotentiator action and an analgesic action (see European Patent Application —Al— 0 096 356). The chemical structure of Arphamenine is expressed by the following formula:

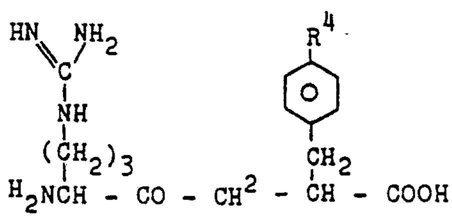

(I)

In the above formula, $R^4$ represents a hydrogen atom or a hydroxyl group. Arphamenine with a hydrogen atom as $R^4$ is named Arphamenine A, and Arphamenine with a hydroxyl group as $R^4$, Arphamenine B.

Now, it has been found that Arphamenine and novel Arphamenine-related compounds can be synthesized easily in good yields when α-amino acid ester derivates and metal salts of substituted or unsubstituted malonic acid diesters are used as starting materials.

Methods of synthesizing compounds which are similar in chemical structure to Arphamenine include the application of the Dakin-West reaction using ketomethylene compounds and oxazolone (Journal of Medicinal Chemistry, Vol. 24, page 964, 1981), and the reaction of Grignard reagents with α-amino acid pyridinethiol esters (Tetrahedron Letter, Vol. 23, page 2533, 1982).

The first method is disadvantageous in preparing the starting ketomethylene compounds, i.e., half-ester-acid chloride compounds of substituted succinic acid, and also has the disadvantage that since the desired compound correspond to compounds of the aforementioned formula (I) having an acylated group, violent acidic hydrolysis is required to return this amino group to the free state. The second method, on the other hand, is disadvantageous in that the starting materials are restricted to a narrow range of compounds, because Grignard reagents are used. Also this method involved in additional step of protecting the ketone group of the compounds of formula (I) in order to avoid possible influences on it which may be exerted when removing the protective group for the functional group.

In contrast, the methods of this invention are simple and efficient. According to this invention there is provided a process for the preparation of compounds of the general formula (I)

$$R{-}CO{-}CH_2{-}\underset{*}{\overset{R_2}{\overset{|}{C}}}H{-}COOH \qquad (I)$$

wherein R represents an α-amino acid residue formed by removing protective groups from an α-amino acid residue having protected functional group and a protected α-amino group, the later α-amino acid residue being described hereinbelow under the designation of $R^1$; $R^2$ represents a hydrogen atom, an alkyl group having 1 to 6 carbon atoms, a phenyl group, or a phenalkyl group the phenyl moiety in both of which is unsubstituted or substituted by hydroxy, nitro or methoxy; and * means that the configuration for the asterisked carbon atom is of the R- of S-type or the R- and S-types combined,
which comprises condensing an α-amino acid derivative of the general Formula (II)

$$R^1{-}COCH_2X \qquad (II)$$

wherein $R^1$ represents an α-amino acid residue corresponding to an α-amino acid molecule from which the α-carboxyl group has been removed, and if the α-amino acid residue contains a functional group in addition to the α-amino group, both the α-amino group and the functional group are protected with usual protective groups, and X represents a bromine or iodine atom, which a substituted or unsubstituted malonic acid diester alkali metal salt of the general formula (III)

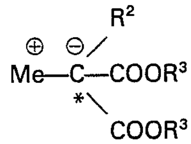

(III)

wherein $R^2$ is as defined above; $R^3$ represents as a carboxyl-protecting group an alkyl group having 1 to 4 carbon atoms or a tetrahydropyranyl group; Me represents an alkali metal; and * is ad defined above, to form a condensation product of the general formula (IV)

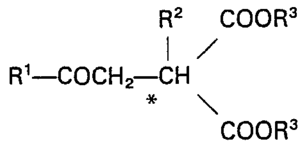

(IV)

wherein $R^1$, $R^2$, $R^3$ and * are as defined above, then removing from the compound obtained one of the $-COOR^3$ groups by a usual decarboxylating method, and finally splitting off the protective groups.

α-Amino acid derivated of the general formula (II), used as the starting material in the process of this invention, are obtained as for example, bromine compounds of the formula (II')

$$R^1-COCH_2Br \quad \text{(II')}$$

by methods which comprise reacting α-amino acids having protected functional groups, expressed by the formula (V)

$$R^1-COOH \quad \text{(V)}$$

wherein $R^1$ is as defined in the general formula (II), with ethyl chloroformate in anhydrous tetrahydrofuran at low temperatures in the presence of triethylamine, and further reacting the system with diazomethane to form diazomethane derivatives of the formula (VI)

$$R^1-COCHN_2 \quad \text{(VI)}$$

and then reacting the compounds of the formula (VI) with an acetic acid solution of hydrobromic acid in tetrahydrofuran. The reaction of the bromine compounds of formula (II') with sodium iodide in acetone gives iodine compounds of the formula (II")

$$R^1-COCH_2I \quad \text{(II")}$$

which are another example of the α-amino acid derivatives of the general formula (II).

Alkali metal salts of malonic acid diesters of the general formula (III), the other reactant in the processes of this invention, can be prepared by reacting substituted or unsubstituted malonic acid diesters of the general formula (VII)

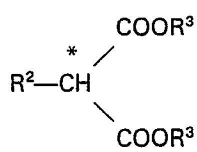

(VII)

wherein $R^2$, $R^3$ and * are as defined in the general formula (III), with alkali metal hydrides, preferably, sodium hydride, in a mixture of N,N-dimethylformamide and hexamethylphosphoric triamide (HMPA).

The condensation of compounds of formula (II) with compounds of formula (III) according to the process of this invention can be carried out by for example reacting both compounds in ordinary organic solvents, such as formamide, dimethylformamide or hexamethylphosphoric triamide (HMPA), for several hours with cooling at 0 to 15°C with ice.

If functional groups, such as a hydroxyl group, an amino group or a carboxyl group, are contained in

the moieties $R^1$ and $R^2$ of the starting compounds for said condensation reaction, these functional groups should desirably be protected when this reaction is carried out.

As protective groups such known in the art can be used for this purpose. Examples of the protective groups for the hydroxyl groups are the tetrahydroparanyl group and the benzyl group. Examples of the protective groups for the amino group are aralkyloxycarbonyl groups, such as a benzyloxycarbonyl group, and alkoxycarbonyl groups, such as a t-butoxycarbonyl group. Protective groups for the carboxyl group are typified by ester-forming groups, such as a tetrahydropyranyl group and alkyl groups.

The condensation reaction between compounds of formula (II) and compounds of formula (III) results in the formation of dicarboxylic acid ester compounds of formula (IV), which are then subjected to a carboxyl- or carboxylate-removing reaction, a reaction for removing one of the $COOR^3$ groups by usual methods. This decarboxylation reaction can be carried out by heating the compounds of formula (IV) in solvents, such as pyridine or dimethylformamide, in the presence of inorganic salts, such as sodium chloride. After the carboxyl- or carboxylate-removing reaction is performed, a reaction for splitting off the protective groups is carried out. For this reaction, various methods known in the art may be used depending on the types of the respective protective groups.

The α-amino acid residue — expressed by $R^1$ — present in the starting compounds of formula (II) in the processes of this invention is derived for example from, the following α-amino acids: Arginine, lysine, phenylalanine, tyrosine, histidine, cysteine, methionine, aspartic acid, glutamic acid, serine, proline, alanine, valine, leucine, isoleucine, and norleucine.

If $R^2$ in the malonic acid diester derivative (III), the other reactant, represents an alkyl group having 1 to 6 carbon atoms, it may be straight-chain or branched. Examples of $R^2$ representing a phenylalkyl group which is substituted or unsubstituted include a benzyl group.

$R^3$ is preferably an alkyl group, such as methyl or ethyl, or the tetrahydropyranyl group.

According to an embodiment of the process of this invention, there is provided a process for preparing Arphamenine A or B of the formula (Ib)

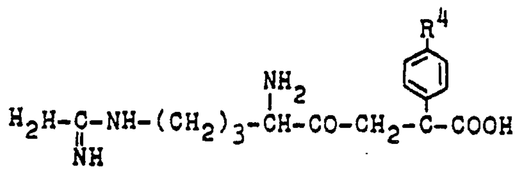

(Ib)

wherein $R^4$ represents a hydrogen atom or the hydroxyl group, which comprises reacting an arginyl-halomethane wherein the amino groups are protected expressed by the formula (IIa)

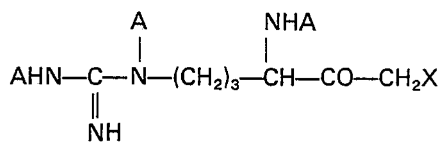

(IIa)

wherein A represents a known amino-protecting group, e.g., an lower alkoxycarbonyl group such as a t-butoxycarbonyl group or an aralkyloxycarbonyl group such as a benzyloxycarbonyl group, and X represents a bromine or iodine atom, with a benzylmalonic acid diester alkali metal salt of the formula (IIIa)

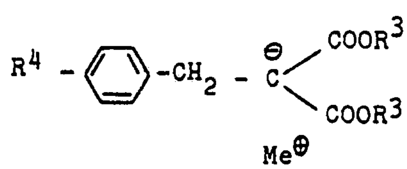

(IIIa)

wherein $R^3$ represents as a carboxyl-protecting group an alkyl group having 1 to 4 carbon atoms or the tetrahydropyranyl group, $R^4$ is as defined above, and Me represents an alkali metal, to form a condensation product of the formula (IVa)

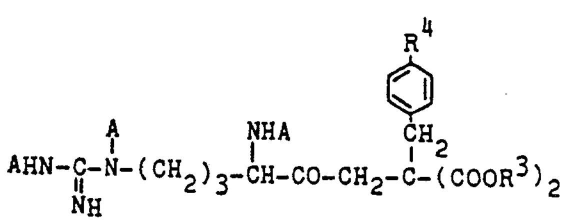

(IVa)

wherein A, $R^3$ and $R^4$ are as defined above, hydrolyzing the resulting product to convert it into a dicarboxylic acid compound of the formula (IVb)

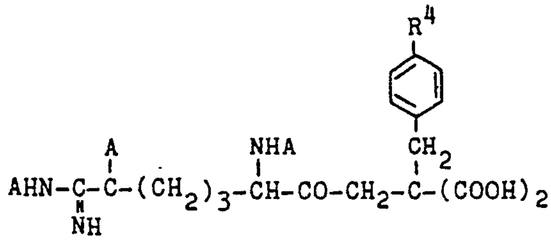

(IVb)

wherein A and $R^4$ are as defined above,
decarboxylating the resulting dicarboxylic acid compound, by removing one of the carboxyl groups from the dicarboxylic acid compound, by a method known in the art to form a monocarboxylic acid compound of the formula (IVc)

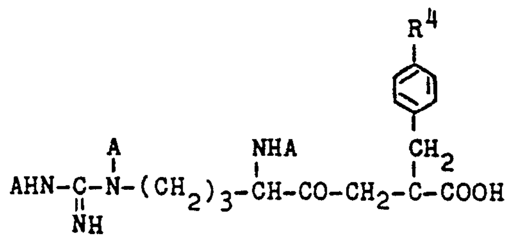

(IVc)

wherein A an $R^4$ are as defined above,
and then subjecting the monocarboxylic acid compound to a reaction for removing the amino-protecting groups (A) by a customary method.

Of the compounds expressed by the general formula I), those other than Arphamenine are novel substances. According to a second gist of this invention, therefore, there are provided Arphamenine-related compounds of the general formula (Ia)

$$R'-COCH_2-\underset{*}{\overset{\overset{\displaystyle R^2}{|}}{C}H}-COOH \qquad (I)$$

wherein R' represents an α-amino acid residue corresponding to an α-amino acid molecule from which the α-carboxy-group has been removed; $R^2$ represents a hydrogen atom, an alkyl group having 1 to 6 carbon atoms, an aryl group or an aralkyl group both of which are unsubstituted or substituted in the nucleus, * means that the configuration for the asterisked carbon atom is of the —R— or S-type or the R- and S-types combined, with the proviso that compounds of the formula I wherein R is an arginine residue corresponding to an arginine molecule from which the α-carboxyl group has been removed, and at the same time, $R^2$ is a benzyl or p-hydroxybenzyl group, are excluded,
and their pharmaceutically acceptable salts.

In the Arphamenine-related compounds expressed by the general formula (Ia), the moity R' —CO— may be any of the following:

A group of the formula

$$H_2N(CH_2)_4-\overset{\overset{\displaystyle NH_2}{|}}{C}H-CO-$$

(derived from lysine);

a group of the formula

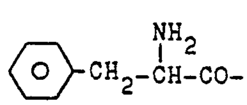

(derived from phenylalanine);

a group of the formula

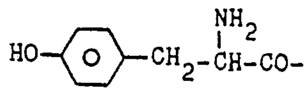

(derived from tyrosine);

a group of the formula

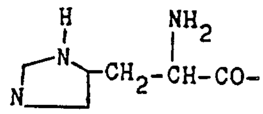

(derived from histidine);

a group of the formula

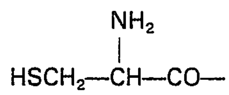

(derived from cysteine);

a group of the formula

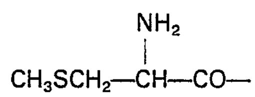

(derived from methionine)

a group of the formula

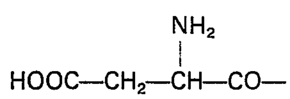

(derived from aspartic acid);

a group of the formula

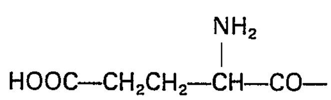

(derived from glutamic acid);

a group of the formula

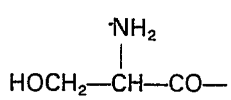

(derived from serine);

a group of the formula

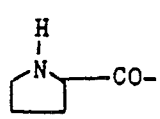

(derived from proline);

a group of the formula

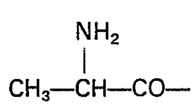

(derived from alanine);

a group of the formula

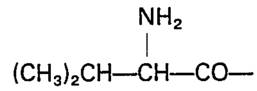

(derived from valine),

a group of the formula

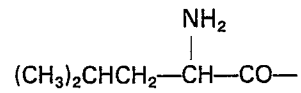

(derived from leucine);

a group of the formula

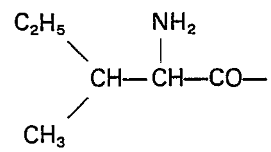

(derived from isoleucine);

a group of the formula

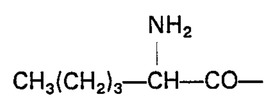

(derived from norleucine).

The pharmaceutically acceptable salts of compounds of the general formulae (I) and (Ia) vary with the acidity or basicity of the molecules of the compounds. Typical examples of the salts acid-addition salts formed between inorganic acids, such as hydrochloric acid or sulfuric acid, and the amino or guanidyl group in the molecules of the compounds. Also exemplified are alkali metal salts formed between alkali metals, such as sodium or potassium, and the carboxyl group in the molecules of the compounds.

The compounds of the general formulae (I) and (Ia) obtained by this invention all have an aminopeptidase B inhibitory activity at a concentration of 0.002 μg/ml to 50 μg/ml. They also show a cell-mediated immunopotentiator activity, like Arphamenine, in tests for their effects on delayed-type hypersentivity.

Hereinbelow, the present invention will be illustrated in more detail with reference to the Referential Examples which show the preparation of the starting materials, and the Examples which show working examples of this invention. This invention, however, is in no way limited to these Examples.

Referential Example 1

(a) Preparation of tri-N-benzyloxycarbonylarginyl-diazomethane

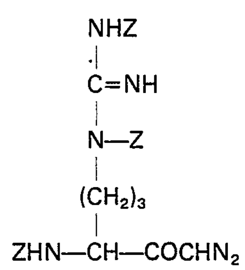

($Z=C_6H_5CH_2OCO—$)

In accordance with the method of Stachowiak et al. (Journal of Medicinal Chemistry, Vol. 22, page 1127, 1979), 3.85 g (6.67 mmoles) of tri-N-benzyloxycarbonyl-arginine (a product of Kokusan Kagaku) and 0.927 ml (6.67 mmols) of triethylamine were suspended in 50 ml of anhydrous tetrahydroforan, and the suspension was cooled to −15°C in a dry ice-acetone bath. 0.638 ml (6.67 mmols) of ethyl chloroformate was added dropwise to the suspension over the course of about 5 minutes with the system stirred and with the reaction temperature maintained at −15°C. The mixture was maintained at the same temperature for an

additional 15 minutes. 20 ml of an ether solution of diazomethane (diazomethane content: about 15 mmols) prepared by a customary method from 2 g of N-nitrosomethylurea was cooled with ice, and added at a time to the above reaction mixture. After the reaction temperature was held at −10°C ± 5°C for about 1 hour, the solvents were evaporated under reduced pressure. The residue was dissolved in 50 ml of ethyl acetate, and the solution was washed with water and with a saturated aqueous solution of sodium bicarbonate. Then, the solution was washed twice with water, and dried over anhydrous sodium sulfate. The dried solution was filtered, and the filtrate was concentrated to about 10 ml. When petroleum ether was added in such an amount as to make the concentrate cloudy, crystals were formed. After the system was allowed to stand overnight at room temperature, the crystals were collected by filtration, and washed with a mixture of ethyl acetate and petroleum ether. 2.498 g (4.32 mmoles) of the captioned compound was obtained as yellow crystals.

Yield: 65%
m.p.: 117.5—118.5°C
IR ($\nu_{max}^{cm^{-1}}$): 2110, 1720—1700
Mass spectrum (FD): m/e 577 (m+1)

(b) Preparation of tri-N-benzyloxycarbonylarginyl-bromomethane

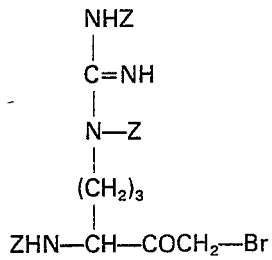

2.450 g (4.08 mmols) of the diazo compound obtained in step (a) was dissolved in 20 ml of tetrahydrofuran, and the solution was cooled with ice. 15 ml of a solution prepared by diluting an acetic acid solution of 25% hydrobromic acid 10-fold with ether was added dropwise to said solution over the course of 3 hours under cooling with ice. The solvents were evaporated under reduced pressure, and the residue was dissolved in 50 ml of ethyl acetate. The solution was sequentially washed with water (twice), and dried over anhydrous magnesium sulfate. After filtration, the filtrates was concentrated to about 7 ml of crystallization. The crystals formed were collected by filtration, and washed with a mixture of ethyl acetate and petroleum ether. After drying, the captioned compound was obtained as colorless needles.

Yield: 80%
m.p.: 136.5—137°C
Mass spectrum (FD): m/e 655 (m+2), 653 (m)
Beilstein's test: Positive
$[\alpha]_D^{25}$: 0°C (c=1.0, chloroform)

(c) Preparation of tri-N-benzyloxycarbonylarginyl-iodo-methane

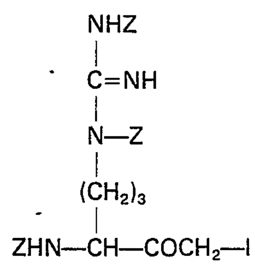

130.7 mg (0.2 mmol) of the bromomethane derivative produced in step (b) and 90 mg (0.6 mmol) of sodium iodide were suspended in 2 ml of acetone, and the suspension was reacted for 3 hours at room temperature at a dark place. Thin-layer chromatography (Merck's silica gel plate Art 5715) of the reaction mixture developed with benzene-acetone (10:1) confirmed the disappearance of the starting material and the appearance of a new spot. Then, the solvent was evaporated under reduced pressure, and the residue was dissolved in 10 ml of ethyl acetate. The solution was washed three times with 7 ml of water wach, and dried over anhydrous magnesium sulfate. After filtration, the filtrate was concentrated to about one-third of the original volume. Ether was added to the concentrate, when crystallization occurred. 96 mg of the primary crystals formed were collected by filtration, and then the filtrate was concentrated, thereby obtaining 30 mg of secondary crystals. Total amount of crystals collected: 126 mg (0.180 mmol).

Yield: 90%
m..: 136—136.5°C
Mass spectrum (FD): m/e 701 (m+1)
Beilstein's test: Positive

(d) Preparation of benzylmalonic acid di-tetrahydropyranyl-ester sodium salt

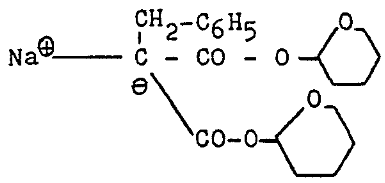

Benzylmalonic acid di-tetrahydropyranyl-ester of the formula

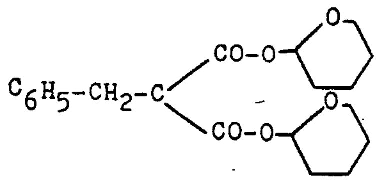

was the first prepared in the following way in accordance with the method of Bowman and Fordham (Journal of Chemical Society, page 3945, 1952):
0.583 g (3 mmols) of benzylmalonic acid and 1.45 ml (16 mmols) of dihydropyran were reacted in 15 ml of benzene under cooling with ice in the presence of a drop of concentrated sulfuric acid as a catalyst. 250 mg of KOH was added to the system, and the mixture was stirred for 30 minutes. The supernatant was taken by decantation, and distilled under pressure in a water bath below 30°C to remove benzene. The desired diester was obtained as a colorless oil.

The resulting oil was dissolved in a mixture of 2 ml of N,N-dimethylformamide and 0.5 ml of hexamethylphosphoric triamide (HMPA). The solution was cooled with ice, and then 36 mg (1.5 mmols) of sodium hydride was added. The mixture was stirred for 1 hour under cooling with ice to form a clear solution containing the captioned sodium salt.

Example 1
Synthesis of Arphamenine

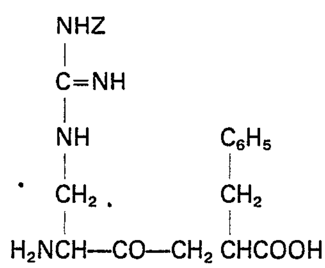

(a) Condensation reaction

To the clear solution containing benzylmalonic acid ditetrahydropyranyl-ester sodium salt prepared in step (d) of Referential Example 1 was added 701 mg (1 mmol) of the tri-N-benzyloxycarbonylarginyl-iodomethane prepared in step (c) of Referential Example 1. The mixture was stirred for 2 hours under cooling with ice, thereby causing a condensation reaction to occur. Thin-layer chromatography on silica gel confirmed the disappearance of the starting iodomethane compound.

(b) Decarboxylation reaction

5 ml of dioxane was added to the reaction mixture of the above step (a), and 1.5 ml of 1N HCl was further added over the course of 2 minutes under cooling with ice. The resulting mixture was stirred for 3 hours under cooling with ice and 2 hours at room temperature to cause hydrolysis, whereby the tetrahydropyranyl groups were removed in the reaction mixture to form a dicarboxylic acid compound of the formula

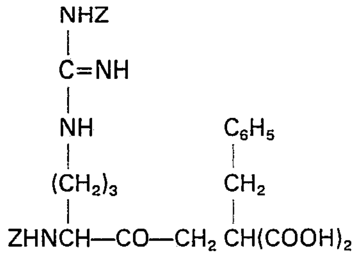

wherein Z is a benzyloxycarbonyl group. (If the hydrolysis reaction mixture is sampled as such and concentrated, followed by adding an ether solution of diazomethane to the concentrated sample in dioxane, then there is formed a dimethyl ester conforming to the separately synthesized products described below in terms of NMR spectrum, thin-layer chromatogram, and mass spectrum (FD) (m/e 794).

Most of dioxane, hydrochloric acid and water were evaporated from said hydrolysis reaction mixture under reduced pressure. 5 ml of pyridine was added to the residue, and the mixture was heated to 100°C, when gases including carbon dioxide gas were generated (decarboxylation reaction). Generation of the gases stopped in about 20 minutes, and thin-layer chromatography (Merck's silica gel plate Art 5715, chloroform-methanol-acetic acid = 10:1:0.5) confirmed the disappearance of the dicarboxylic acid and the formation of two new spots.

Pyridine and N,N-dimethylformamide were distilled away from the reaction mixture under reduced pressure. The residue was dissolved in 15 ml of ethyl acetate, and the solution was washed three times with 10 ml of water each. Then, the solution was dried over anhydrous magnesium sulfate, and filtered. 100 ml of activated carbon was added to the filtrate which was filtered again.

The resulting filtrate was concentrated under reduced pressure to obtain the desired tri-N-benzyloxycarbonyl-Arphamenine A as oily matter. This product was dissolved in 2 ml of chlorform, and the solution was adsorbed onto a silica gel column (CC-7, a product of Mallinckrodt Inc., 35 g). The column was developed with chloroform-methanol (120:1) to obtain fractions with a volume of 3.5 ml each. Fractions Nos. 23 to 26 gave 352 mg of a compound corresponding to a spot with a high Rf value, and Fractions Nos. 39 to 42, 67 mg of a compound corresponding to a spot with a low Rf value. The remaining fractions, Nos. 27 to 38, gave 212 mg of a mixture. Said compound with the high Rf value is designated as (Va), and the compound with the low Rf value, as (Vb). Their physical constants are shown below.

Compound (Va)

$^1H$—NMR (δ, 80 MHz, in $CDCl_3$):
8.26 (=NH), 7.23 ($C_6H_5$),

5.18, 5.11, 5.01 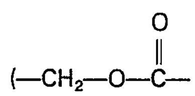 each),

4.63 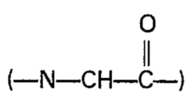

Methyl ester of the compound (Va) that was obtained by treating this compound with diazomethane showed a mass spectrum (FD) of m/e 738 (m+1).

Compound (Vb)

$^1H$—NMR (δ, 80 MHz, in $CDCl_3$):
9.25 (=NH), 7.31, 7.28, 7.24 ($C_6H_5$),

5.18, 5.08, 5.03 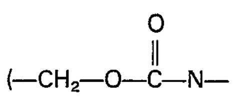 each),

4.55 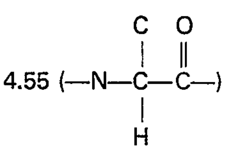

Methyl ester of the compound (Vb) that was obtained by treating this compound with diazomethane showed a mass spectrum (FD) of m/e 738 (m+1).

(c) Deprotection reaction (formation of Arphamenine A)

100 mg of the tri-N-benzyloxycarbonyl-Arphamenine (Va) or (Vb) obtained in step (b) was dissolved in a mixture of 3 ml of dioxane, 0.5 ml of distilled water, and 0.3 ml of 1N hydrochloric acid. The solution was catalytically reduced for 5 hours in an atmosphere of hydrogen at 3 atmospheric pressure in the presence of 10 mg of palladium black as a catalyst. Thin-layer chromatography (Merck's silica gel plate Art 5715, developed with phenol-water (3:1)) of the reaction mixture confirmed the disappearance of the starting material and the formation of a spot with the same Rf value as for Arphamenine. Then, the catalyst was removed by filtration, and the solvent and water were evaporated under reduced pressure. The resulting crude powder of Arphamenine chloride was dissolved in 0.1 ml of water, and the solution was adsorbed onto 4 ml of a nonionic adsorption resin, Diaion HP-20. The resin was developed with water to obtain fractions with a volume of 0.4 ml each. The desired Arphamenine was given in Fractions Nos. 12 to 28. These active fractions were combined, and concentrated under reduced pressure to about 1 ml. About 0.2 ml of Dowex WGR was added to the concentrate, which was then adjusted to neutral pH and filtered. Dowex resin was washed with water, and the waschings were combined with the filtrate. The combined liquid was concentrated, and lyophilized to obtain 23 mg of the desired Arphamenine A.

Yield: 46.4%

The desired Arphamenine obtained from said compound (Va) showed, on thin-layer chromatography, the same Rf values as that of naturally occuring Arphamenine obtained by a fermentation process. This Arphamenine from the compound (Va) is designated as Substance (I'). The corresponding substance from the aforementioned compound (Vb) is designated as Substance (I''). The physical properties of these substances will be mentioned below.

Substance (I')

$^1H$—NMR (δ, 80 MHz, in heavy water):

| | |
|---|---|
| 7.80—7.98 ($C_6H_5$), | 4.83 (CH) |
| 3.96—3.84 (CH, $CH_2$), | 3.34—3.68 ($CH_2 \times 2$), |
| 2.30—2.70 ($CH_2$), | 2.01—2.30 ($CH_2$) |

Mass spectrum (SIMS): m/e 321 (m+1)
:(FD): m/e 321 (m+1)

Sakaguchi's reaction, ninhydrin reaction: Positive Aminopeptidase B inhibitory activity ($IC_{50}$): 0.007 mcg/ml (naturally occuring Arphamenine A: 0.005 mcg/ml)

Substance (I'') (optical isomer)

$^1H$—NMR (δ, 80 MHz, in heavy water):

| | |
|---|---|
| 7.78—7.86 ($C_6H_5$), | 4.86 (CH), |
| 3.72—3.85 (CH, $CH_2$), | 3.35—3.67 ($CH_2 \times 2$), |
| 2.35—2.70 ($CH_2$), | 2.01—2.30 ($CH_2$) |

Mass spectrum (SIMS): m/e 321 (m+1)
(FD): m/e 321 (m+1)

Sakaguchi's reaction, ninhydrin reaction: Positive Aminopeptidase B inhibitory activity ($IC_{50}$) described above were determined by an improvement of the Hopsu method disclosed in the specification of European Patent Application No. A1—0096 356 (this applies to the disclosure of the Examples to follow).

## Example 2

Synthesis of 5-amino-8-guanidyl-4-oxo-1-octanoic acid

$NH_2$
|
C=NH
|
NH
|
$(CH_2)_3$
|
$H_2NCH—CO—CH_2CH_2COOH$

7.01 mg (1 mmol) of tri-N-benzyloxycarbonyl-arginyl-iodo-methane obtained in step (c) of Referential Example 1 was condensed with malonic acid di-tetrahydropyranyl-ester sodium salt (1.1 mmols) that had been prepared by the reaction of malonic acid di-tetrahydropyranyl-ester with sodium hydride in the same way as in step (d) of Referential Example 1. The same procedure as in step (a) of Example 1 was applied to this condensation reaction. The resulting condensation product was reacted, treated and purified in the same way as in the steps (b) and (c) of Example 1 to obtain the captioned compounds as a monohydrochloride.

Yield: 72.3 mg (27%)
Mass spectrum (SIMS): m/e 231 (m+1)
Sakaguchi's reaction, ninhydrin reaction: Positive

Example 3

Synthesis of 5-amino-8-guanidyl-4-oxo-2-phenyl-1-octanoic acid

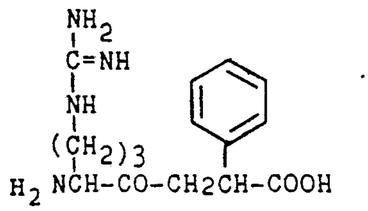

Tri-N-benzyloxycarbonyl-arginyl-iodomethane obtained in step (c) of Referential Example 1 was condensed with phenylmalonic acid di-tetrahydropyranyl-ester sodium salt that had been prepared by the reaction between phenylmalonic acid di-tetrahydropyranyl-ester and sodium hydride in the same was as in the step (d) of Referential Example 1. The same procedure as in the step (a) of Example 1 was used for this condensation reaction. The resulting condensation product was reacted, treated and purified in the same way as in the steps (b) and (c) of Example 1. The captioned compound was obtained as a monohydrochloride.

Mass spectrum (SIMS): m/e 307 (m+1)
(FD): m/e 307 (m+1)

Sakaguchi's reaction, ninhydrin reaction: Positive Aminopeptidas B inhibitory activity ($IC_{50}$): 0.045 mcg/ml

Example 4

Synthesis of 5-amino-8-guanidyl-4-oxo-2-methyl-1-octanoic acid

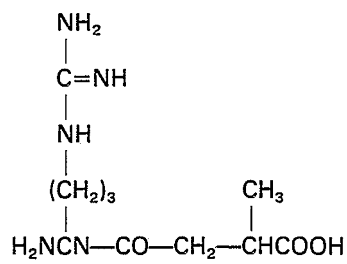

Tri-N-benzyloxycarbonyl-arginyl-iodomethane obtained in the step (c) of Referential Example 1 was condensed with methyl-malonic acid di-tetrahydropyranol-ester sodium salt which had been prepared by the reaction between methylmalonic acid di-tetrahydropyranyl-ester with sodium hydride in the same way as in step (d) of Referential Example 1. The same procedure as in step (a) of Example 1 was used for this condensation reaction. The resulting condensation product was reacted, treated and purified in the same was as in the steps (b) and (c) of Example 1 to obtain the captioned compound as a monohydrochloride.

Mass spectrum (SIMS): M/e 245 (m+1)
Aminopeptidase B inhibitory activity ($IC_{50}$): 0.081 mcg/ml

Referential Example 2

(a) N-benzyloxycarbonyl-phenylalanine was reacted with diazomethane and then with an acetic acid solution of hydrobromic acid in the same was as in the steps (a) and (b) of Referential Example 1 to obtain N-benzyloxycarbonyl-phenylalanyl-bromomethane of the formula

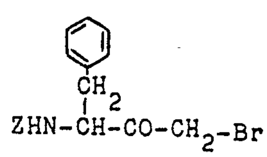

wherein Z represents a benzyloxycarbonyl group.

Yield: 47%
m.p.: 102.5—103°C
$[\alpha]_D$: + 21° (c=0.6, $CHCl_3$)
Mass spectrum (FD): m/e 378 (m+2), 376 (m)

(b) 1.58 g (10.8 mmols) of methylmalonic acid dimethyl ester was dissolved in a mixture of 6 ml of N,N-dimethylformamide and 0.6 ml of hexamethylphosphoric triamide. The solution was cooled with ice, and then 230 mg (9.58 mmols) of sodium hydride was added, followed by stirring the mixture for 1 hour. A clear solution containing methylmalonic acid dimethyl ester sodium salt of the formula

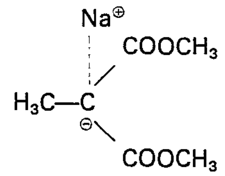

was obtained.

Example 5

Synthesis of 5-amino-2-methyl-4-oxo-6-phenyl-1-hexanoic acid

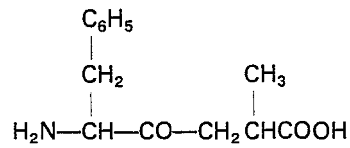

(a) Formation of 5-benzyloxycarbonylamino-2-methoxycarbonyl-2-methyl-4-oxo-6-phenylhexanoic acid methyl ester

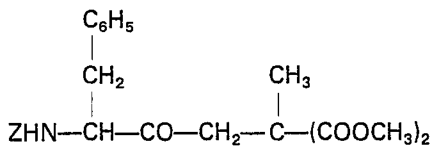

(Z is a benzyloxycarbonyl group)

2.26 g (6.0 mmols) of N-benzyloxycarbonyl-phenylalanyl-bromomethane obtained in step (a) of Referential Example 2 was added to the solution of methylmalonic acid dimethyl ester sodium salt obtained in the step (b) of Referential Example 2. The mixture was stirred for 30 minutes under cooling with ice. Thin-layer chromatography on silica gel (Merck's TLC plate, Art 5715, developed with benzene-acetone = 10:1) of the reaction mixture confirmed the disappearance of starting bromomethane compound and the appearance of a new spot. N,N-dimethylformamide was evaporated from the reaction mixture under reduced pressure. The residue was dissolved in 40 ml of ether, and the solution was washed with water, 0.05 N hydrochloric acid, an aqueous solution of sodium bicarbonate, and water (twice) in this sequence. Then, the washed solution was dried over anhydrous sodium sulfate, and filtered. The solvent was evaporated from the filtrate to obtain 2.263 g of a pale yellow oil. This matter was purified by silica gel chromatography (Wako's C—200, 64 g, developer: benzene-acetone = 30:1) to give 1.75 g (3.97 mmols) of the captioned compound as oily matter.

Yield: 66%

$^1H$—NMR (δ, 60 MHz, in $CDCl_3$): 7.30 ($C_6H_5$), 7.22 ($C_6H_5$), 5.37 (NH, doubled, J=8HZ), 5.05 (—$CH_2O$),

4.72—4.40 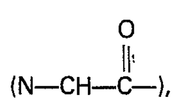,

3.65 (—$OCH_3$), 3.17—2.90 ($CH_2$ × 2), 1.43 (—$CH_3$)

Mass spectrum (FD): m/e 442 (m+1)

(b) Formation of 5-benzyloxycarbonylamino-2-methyl-4-oxo-6-phenyl-hexanoic acid methyl ester

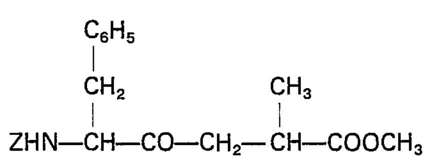

In accordance with the method of A. P. Krapcho et al. (Synthesis, 805 (1982)), 1.70 g of the product obtained in the above step (a) was dissolved in a mixture of 17 ml of N,N-dimethylformamide and 0.18 ml

of water, 450 mg of sodium chloride was added, and the mixture was heated at 150°C for 32 hours, thereby removing one of the methoxycarbonyl groups. The solvents were evaporated under reduced pressure, and the residue was separated into different layers with the addition of ethyl acetate and 50 ml of water. The separated system was further washed with 50 ml of water each. The organic layer was dried over anhydrous magnesium sulfate, and filtered. The filtrate was concentrated to dryness, and the resulting oily substance was purified by silica gel column chromatography (silica gel: Wako C—200, developer: benzene-acetone = from 80:1 to 40:1, volume of each fraction: 6 ml) Fractions Nos. 34 to 48 were combined, and concentrated to dryness to obtain 314 mg (0.82 mmol) of the captioned compound as an oily substance.

Yield: 19%

$^1$H—NMR (δ ppm, 80 MHz, in $CDCl_3$): 0.96—1.50 ($CH_3$), 2.65—3.43 ($CH_2$, $CH_2$, CH), 3.63 ($CH_3O$—, 3.64 ($CH_3O$—), 4.38—4.65 (CH), 5.03 ($CH_2$), 5.05 ($CH_2$), 5.20—5.45 (NH)

This $^1$H—NMR pattern showed the resulting product to be a mixture (about 1:1) of two diastereomers.

Mass spectrum (FD): m/e 384 (m+1)

(c) Formation of 5-benzyloxycarbonylamino-2-methyl-4-oxo-6-phenyl-1-hexane

200 mg (0.52 mmol) of the methyl ester product obtained in above step (b) was dissolved in 2 ml of methanol, and the solution was cooled with ice. Then 0.5 ml of 1N NaOH was added, and the mixture was stirred for 6 hours under cooling with ice. After silica gel thin-layer chromatography confirmed the disappearance of the starting methyl ester compound, methanol was evaporated under reduced pressure in a water bath at 30°C. 3 ml of water and 3 ml of ether were added to the residue to separate it into different layers. The aqueous layer was further extracted twice with ether, and cooled with ice. About 0.6 ml of 1N HCl was added the pH to 2, and the mixture was extracted twice with ethyl acetate. The organic layer was washed twice with a saturated aqueous solution of sodium chloride, and dried over anhydrous magnesium sulfate. The dried liquid was filtered, and the solvent was evaporated from the filtrate under reduced pressure to obtain 140 mg (0.38 mmol) of the captioned compound as an oily substance.

Yield: 73%

$^1$H—NMR (δ ppm, 60 MHz, in deuterochloroform): 0.97—1.32 ($CH_3$), 2.60—3.27 ($CH_2$, $CH_2$, CH), 4.37—4.72 (CH), 5.03 ($CH_2$), 5.17—5.45 (NH), 6.95—7.33 ($C_6H_5$)

Mass spectrum (FD): m/e 370 (m+1), 254, 115

(d) Formation of 5-amino-2-methyl-4-oxo-6-phenyl-1-hexanoic acid

92.4 mg (0.25 mmol) of the product obtained in the above step (c) was dissolved in a mixture of 1 ml of dioxane, 0.2 ml of water, and 30 ml of 1N HCl. A palladium catalyst was added to the solution, and the mixture was catalytically reduced in an atmosphere of hydrogen at 3 atmospheric pressure to remove the benzyloxycarbonyl group. Two hours later, thin-layer chromatography (Merck's TLC plate Art 5715, developer: chloroform-methanol-acetic acid = 10:1:0.5) of the reaction mixture confirmed the disappearance of the starting compound having a protected amino group, and the appearance of a spot showing a positive ninhydrin reaction. The catalyst was removed by filtration, and the filtrate was concentrated to dryness. The residue was dissolved in 0.15 ml of water, and the solution was adsorbed onto a 3 ml column of Diaion HP—20. The column was washed with 6 ml of water, and developed with a 10% aqueous solution of methanol to elute the desired compound. The eluate was adjusted to a pH of 6 with Dowex WGR (OH type), and filtered. The filtrate was concentrated as a colorless powder.

Yield: 75%

$^1$H—NMR (δ ppm, 60 MHz, in methanol-$D_4$): 1.02—1.34 ($CH_3$), 2.65—3.24 ($CH_2$, $CH_2$, CH), 4.56—4.75 (CH), 7.05—7.38 ($C_6H_5$)

Mass spectrum (FD): m/e 236 (m+1)

Aminopeptidase B inhibitory activity ($IC_{50}$): 48 mcg/ml

Reference Example 3

(a) 4.05 g (18.8 mols) of N-t-butoxycarbonyl-proline was reacted with ethyl chloroformate and diazomethane in the same way as in step (a) of Referential Example 1 to obtain N-t-butoxycarbonyl-prolyl-diazomethane of the formula

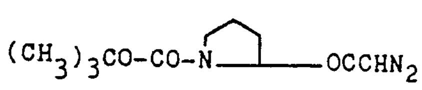

as a yellow oil.

Yield: 4.25 g (94%)

IR (film method, neat): $\nu_{max}^{cm^{-1}}$ 2108

An acetic acid ether solution of 2.5% hydrobromic acid was added dropwise to a tetrahydrofuran solution of 3.93 g of the so obtained N-t-butoxycarbonyl-prolyl-diazomethane under cooling with ice in the same way as in step (b) of Referential Example 1. The reaction mixture was concentrated to dryness, and the residue was extracted. The extract was purified by silica gel chromatography (silica gel: Wako C200, 120 g developer: benzene-ethyl acetate = 17.1 to 13.1) to obtain 2.98 g of N-t-butoxycarbonyl-propyl-brom-methane of the formula

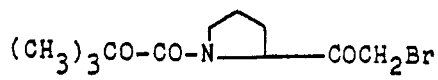

as a yellow oil.

Yield: 62%

Mass spectrum (FD): m/e 294 (m+2), 292 (m)

(b) 650 mg of isobutylmalonic acid dimethyl ester was reacted with 48 mg of sodium hydride in the same way as in step (d) of Referential Example 1 to obtain a solution of isobutylmalonic acid diethyl ester sodium salt.

Example 6

Synthesis of 2-isobutyl-4-(2-pyrrolidyl)-4-oxo-1-butanoic acid

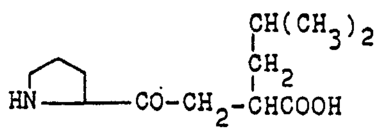

(a) Formation of 2-isobutyl-2-ethoxycarbonyl-4-(1′-t-butoxycarbonyl-2′-pyrrolidyl)-4-oxo-1-butanoic acid ethyl ester

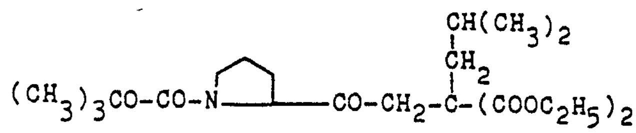

270 mg of the N-t-butoxycarbonyl-prolyl-brommethane obtained in the step (a) of Referential Example 3 was subjected to a condensation reaction with the solution of isobutylmalonic acid diethyl ester sodium slat obtained in the step (a) of Referential Example 3. The procedure of Example 1 was used for this condensation reaction. The condensation product was purified by silica gel column chromatography to obtain 172 mg of the captioned compound as a colorless oil.

Yield: 44%

$^1$H—NMR (δ ppm, in deuterochloroform): 0.78—0.89 ($CH_3$), 1.1—1.33 ($CH_3$), 1.42 ($(CH_3)_3$), 1.55—2.10 ($CH_2$, $CH_2$), 3.03—3.28 ($CH_2$), 3.30—3.67 ($CH_2$), 3.92 —4.28 ($CH_2$)

Mass spectrum (FD): m/e 428 (m+1), 257, 170

(b) Formation of 2-isobutyl-4-(1-t-butoxycarbonyl-2-pyrrolidyl)-4-oxo-butanoic acid ethyl ester

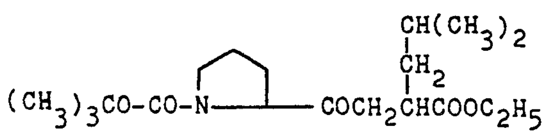

428 mg (1 mmol) of the product obtained in the above step (a) was dissolved in a mixture of 3 ml of DMSO and 0.1 ml of water, 120 ml of sodium chloride was added to the solution, and the mixture was heated at 160°C for 4 hours to remove one of the ethoxycarbonyl groups. The resulting product was treated in the same way as in step (b) of Example 1, and purified by slica gel column chromatography to obtain 193 mg of the captioned compound as an oily substance.

Yield: 54%

Mass spectrum (FD): m/e 356 (m+1), 185, 170

(c) Formation of 2-isobutyl-4-(1-t-butoxycarbonyl-2-pyrrolidyl)-4-oxo-butanoic acid

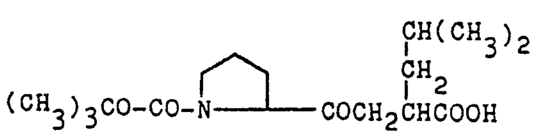

182 mg of the ethyl ester produced in step (b) was hydrolyzed with an alkali in the same way as in the step (c) of Example 5. The reaction mixture was concentrated, and then 5 ml of ethyl acetate was added to the concentrate. 0.48 ml of 1N hydrochloric acid and 3 ml of water were added to the mixture to separate it

into different layers. The organic layer was washed twice with a saturated aqueous solution of sodium chloride, and dried over anhydrous magnesium sulfate. Then, the layer was filtered, and the filtrate was concentrated to dryness to obtain 164 mg of an oily substance. This substance was purified by silica gel column chromatography (silica gel: CC—7, a product of Mallinckrodt Inc., 5 g developer: chloroform-methanol = 20:1) to obtain 128 mg of the captioned compound as a colorless oil.

Yield: 76%

Mass spectrum (FD): m/e 328 (m+1)

(d) Formation of 2-isobutyl-4-(2-pyrrolidyl)-4-oxo-1-butanoic acid

120 mg of the product of step (c) was dissolved in 3 ml of dioxane, and the solution was cooled with ice. 2.4 ml of a dioxane solution containing 6N hydrochloric acid was added, and the mixture was stirred for 20 hours under cooling with ice to remove the protective group.

Thin-layer chromatography (silica gel as thin layer: Merck's TLC Art 5715, developer: chloroform-methanol-acetic acid = 10:1:0.5) of the reaction system confirmed the disappearance of the starting t-butoxycarbonyl compound and the appearance of a new spot showing a positive ninhydrin reaction. The solvent used in the reaction was evaporated under reduced pressure, and the residue was treated, purified and neutralized in the same way as in step (d) of Example 5. 52 mg of the captioned compound was obtained as an amorphous powder.

Yield: 62%

(d) Formation of 2-isobutyl-4-(2-pyrrolidyl)-4-oxo-1-butanoic acid

120 mg of the product of step (c) was dissolved in 3 ml of dioxane, and the solution was cooled with ice. 2.4 ml of a dioxane solution containing 6N hydrochloric acid was added, and the mixture was stirred for 20 hours under cooling with ice to remove the protective group. Thin-layer chromatography (silica gel as thin layer: Merck's TLC Art 5715, developer: chloroform-methanol-acetic acid = 10:1:0.5) of the reaction system confirmed the disappearance of the starting t-butoxycarbonyl compound and the appearance of a new spot showing a positive ninhydrin reaction. The solvent used in the reaction was evaporated under reduced pressure, and the residue was treated, purified and neutralized in the same way as in step (d) of Example 5. 52 mg of the captioned compound was obtained as an amorphous powder.

Yield: 62%

m.p.: 80—105°C

$^1$H—NMR (δ, 60 MHz, in Methanol-$D_4$): 0.80—0.92 ($CH_3$), 1.47—2.03 ($CH_2$, $CH_2$, CH), 2.98—3.24 ($CH_2$), 3.40—3.52 ($CH_2$), 4.68—4.84 (CH)

Mass spectrum (FD): m/e 228 (m+1)

Aminopeptidase B inhibitory activity ($IC_{50}$): 12 mcg/ml

Referential Example 4

(a) N-benzyloxycarbonyl-asparagine benzyl ester was reacted wth ethyl chloroformate and diazomethane in the same way as in step (a) of Referential Example 1 to obtain N-benzyloxycarbonyl-asparaginyl-diazomethane ß-benzyl ester of the formula

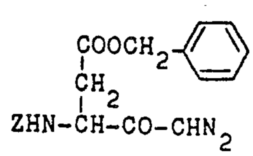

(Z is a benzyloxycarbonyl group)

as a pale yellow solid.

m.p.: 120—125°C

Yield: 67%

IR ($\nu_{max}^{cm^{-1}}$): 2.03

Mass spectrum (FD): m/e 382 (m+1)

(b) The diazo compound produced in step (a) was reacted with hydrobromic acid in the same way as in step (b) of Referential Example 1 to obtain N-benzyloxycarbonyl-asparaginyl-bromethane ß-benzyl ester of the formula

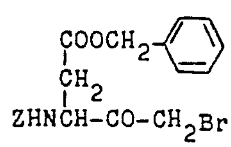

as colorless crystals.

Yield: 89%
Mass spectrum (FD): m/e 436 (m+2)
m/e 434 (m)

## Example 7
### Synthesis of 5-amino-2-isopropyl-4-oxo-heptanedicarboxylic acid

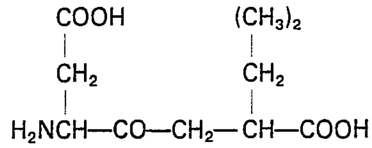

(a) The N-benzyloxycarbonyl-asparaginyl-bromomethane ß-benzyl ester obtained in step (b) of Referential Example 4 was reacted with isopropylmalonic acid diethyl ester sodium salt in the same way as in step (a) of Example 1. The reaction mixture was treated in the same way as in step (a) of Example 1 to obtain a compound of the formula

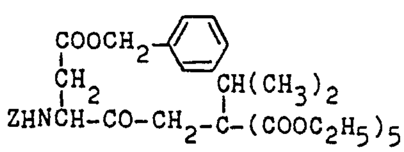

as a colorless oil.
Yield: 63%
Mass spectrum (FD): m/e 556 (m+1), 312, 243

(b) The product obtained in above step (a) was treated in the same way as in step (b) of Example 5 to remove the ethoxycarbonyl group, thereby obtaining a compound of the formula

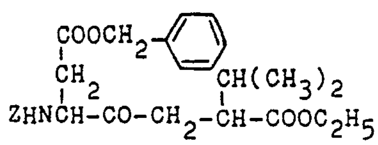

as a colorless oil.
Yield: 67%
Mass spectrum (FD): m/e 484 (m+1)

(c) The product obtained in step (b) was hydrolyzed with an alkali in the same way as in step (c) of Example 5 to obtain a compound of the formula

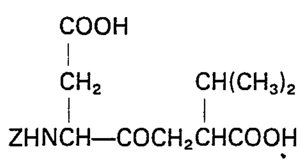

as a colorless amorphous solid (m.p. 183—198°C).
Yield: 82%
Mass spectrum (FD): m/e 366 (m+1)
A dimethyl ester obtained by treating the resulting compound with diazomethane showed a mass spectrum of m/e 394 (m+1).

(d) The product obtained in the above step (c) was catalytically reduced in the same way as in step (d) of Example 5 to remove the benzyloxycarbonyl group, thereby obtaining the desired 5-amino-2-isopropyl-4-oxoheptadicarboxylic acid as a colorless solid (m.p. 195—220°C).
Yield: 86%
Mass spectrum (FD): m/e 232 (m+1)

Example 8
Synthesis of 6-mercaptò-5-amino-4-oxo-2-methyl-heptanoic acid

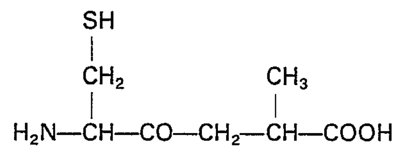

N-di-t-butoxycarbonyl-cysteinyl-brommethane derived from N-di-t-butoxycarbonylcysteine was reacted with methylmalonic acid diethyl ester sodium salt in the same way as in step (a) of Example 5. The reaction product was treated in the same way as in the steps (b), (c) and (d) of Example 5 to obtain the captioned compound as a pale yellow solid (m.p. 124—137°C).

Mass spectrum (FD): m/e 193 (m+2), 191 (m)

Aminopeptidase B inhibitory activity ($IC_{50}$): 0.13 mcg/ml

**Claims**

1. A process for the preparation of compounds of the formula I

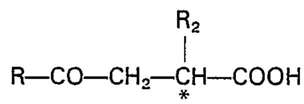
(I)

wherein R represents an α-amino acid residue corresponding to an α-amino acid molecule from which the α-carboxyl group has been removed, $R^2$ represents a hydrogen atom, an alkyl group having 1 to 6 carbon atoms, a phenyl- or phenylalkyl group the phenyl moiety in both of which is unsubstituted or substituted by hydroxy, nitro or methoxy and * means that the configuration at the asterisked carbon atom is of the R- and S-type or the R- and S-types combined, characterized by condensng an α-amino acid derivative of the formula II

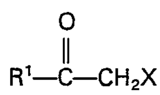
(II)

wherein $R^1$ has the same meaning as R above with the proviso that, if the α-amino acid residue contains a functional group in addition to the α-amino group, both the α-amino group and the functional group are protected with usual protective groups, and X represents bromine or iodine, with a substituted or unsubstituted malonic acid diester alkyli metal salt of the formula III

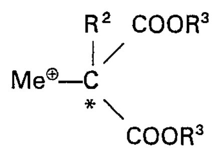
(III)

wherein $R^2$ is as defined above; $R^3$ represents a carboxyl-protecting group and Me represents an alkali metal; and * has the meaning as defined above, to form a compound of the formula

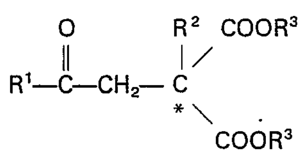

wherein $R^1$, $R^2$ and $R^3$ are as defined above, removing one of the $—COOR^3$ grups by a decarboxylating reaction known in the art, and finally splitting off the protective groups by methods known in the art.

2. A process as defined in claim 1 characterized in that $R^3$ is $C_1$—$C_4$-alkyl or tetrahydropyranyl.

3. A process as defined in claim 1 characterized in that $R^2$ is hydrogen, an alkyl group having 1 to 6 carbon atoms, phenyl or benzyl, both of which may be unsubstituted or substituted in the nucleus by hydroxy, nitro or methoxy, and R is a group of the formulae

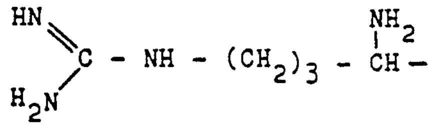

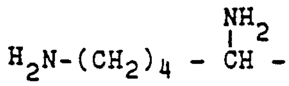

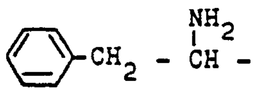

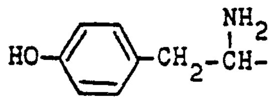

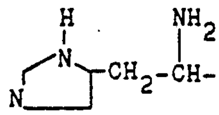

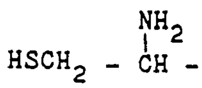

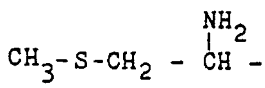

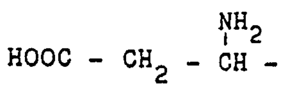

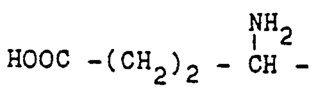

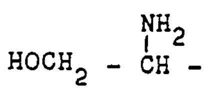

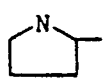

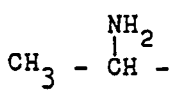

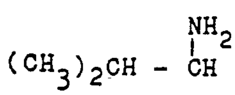

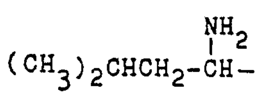

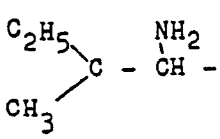 or 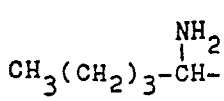

4. A process as defined in claim 1—3 characterized in that the condensation reaction is carried through in a solvent and at a temperature of from 0 to 15°C.

5. A compound of the formula I

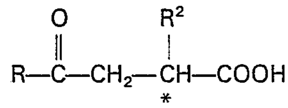

wherein R, $R^2$ and * have the meaning as defined in claim 1 with the proviso that R is not an arginine residue corresponding to an arginine molecule from which the α-carboxyl group has been removed if $R^2$ is the benzyl group or a p-hydroxybenzyl group, and the pharmaceutically acceptable salts thereof.

6. A compound of the formula I

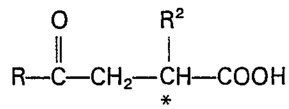

(I)

wherein R, $R^2$ and * have the meaning as defined in claim 3 with the proviso that R is not a group of the formula

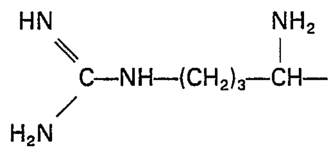

if $R^2$ is the benzyl group or a p-hydroxybenzyl group, and the pharmaceutically acceptable salts thereof.

7. A pharmaceutical composition comprising as active ingredient a compound of the formula I as claimed in claims 5 and 6 or a physiologically acceptable salt thereof in association with a pharmaceutically acceptable carrier.

8. A compound of the formula I or a physiologically acceptable salt thereof for use as a medicament.

**Patentansprüche**

1. Ein Verfahren zur Herstellung von Verbindungen der Formel I

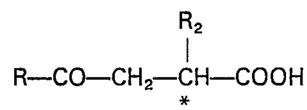

(I)

worin R einem α--Aminosäurerest bedeutet, der einem α-Aminosäuremolekül entspricht, aus dem die α-Carboxygruppe entfernt wurde, $R^2$ für ein Wasserstoffatom, eine Alkylgruppe mit 1 bis 6 Kohlenstoffatomen oder eine Phenyl- oder Phenylalkylgruppe steht worin der Phenylrest nicht substituiert oder durch Hydroxy, Nitro oder Methoxy substituiert sein kann und * bedeutet, daß es sich bei der Konfiguration des mit dem Sternchen versehenen Kohlenstoffatoms um eine vom R- oder S-Typ oder um eine Kombination beider handelt, dadurch gekennzeichnet, daß man ein α-Aminosäurederivat der Formel II

$$R^1—COCH_2X \qquad (II)$$

worin $R^1$ dieselbe Bedeutung wie R oben hat, unter der Voraussetzung, daß, falls der α-Aminosäurerest zusätzlich zur α-Aminogruppe eine funktionelle Gruppe enthält, sowohl die α-Aminogruppe als auch die funktionelle Gruppe mit den üblichen Schutzgruppen geschützt sind, und X für Brom oder Jod steht, mit einem nicht-substituierten oder substituierten Malonsäurediesteralkalimetallsalz der Formel III

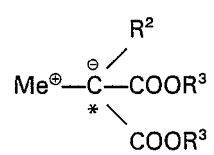

(III)

worin $R^2$ die obige Bedeutung hat, $R^3$ eine Carboxylschutzgruppe bedeutet, Me für ein Alkalimetall steht und * die obige Bedeugunt besitzt, zu einer Verbindung der Formel

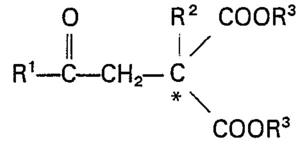

worin $R^1$, $R^2$ und $R^3$ die obige Bedeutung besitzen, kondensiert, eine der —$COOR^3$— Gruppen durch eine übliche Decarboxylierungsreaktion entfernt und schließlich die Schutzgruppen durch ebenfalls übliche Methoden abtrennt.

2. Ein Verfahren gemäß Anspruch 1, dadurch gekennzeichnet, daß $R^3$ $C_1$—$C_4$ Alkyl oder Tetrahydropyranyl bedeutet.

3. Ein Verfahren gemäß Anspruch 1, dadurch gekennzeichnet, daß $R^2$ Wasserstoff, eine Alkylgruppe mit 1 bis 6 Kohlenstoffatomen, Phenyl oder Benzyl bedeutet, wobei die beiden letzteren im Kern nichtsubstituiert oder durch Hydroxy, Nitro oder Methoxy substituiert sein können und R eine Gruppe der Formeln

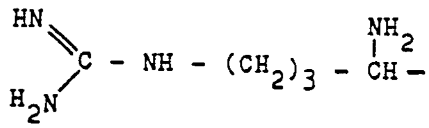

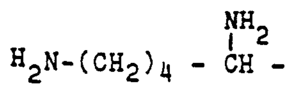

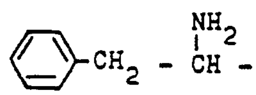

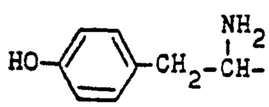

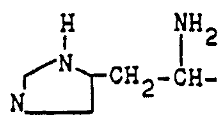

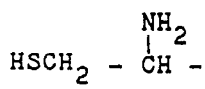

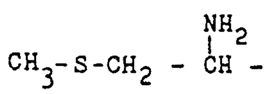

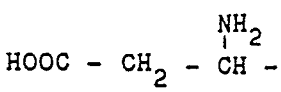

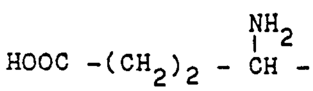

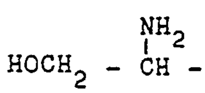

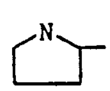

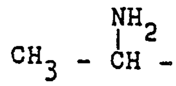

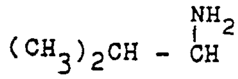

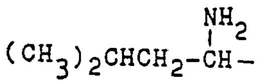

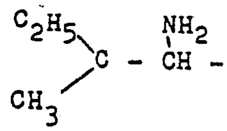

oder

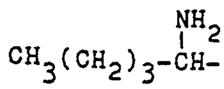

4. Ein Verfahren gemäß Anspruch 1 bis 3, dadurch gekennzeichnet, daß die Kondensierungsreaktion in einem Lösungsmittel bei einer Temperatur von 0 bis 15°C erfolgt.

5. Eine Verbindung der Formel I

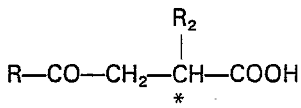

(I)

worin R, $R^2$ und * die im Anspruch 1 angegebene Bedeutung besitzen, unter der Voraussetzung, daß falls $R^2$ die Benzylgruppe oder die p-Hydroxybenzylgruppe bedeutet, R nicht für einen Argininrest steht, der einem Argininmolekül entspricht, aus dem die α-Carboxylgruppe entfernt wurde, sowie deren pharmazeutisch verträgliche Salze.

6. Eine Verbindung der Formel I

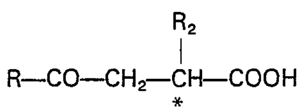

(I)

worin R, $R^2$ und * die im Anspruch 3 angegebene Bedeutung besitzen, unter der Voraussetzung, daß R nicht eine Gruppe der Formel

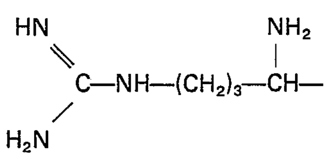

bedeutet, falls $R^2$ für die Benzylgruppe oder eine p-Hydroxybenzylgruppe steht, sowie deren pharmazeutisch verträgliche Salze.

7. Ein pharmazeutisches Präparat enthaltend als Wirkstoff eine Verbindung der Formel I gemäß Anspruch 5 und 6 oder ein pharmazeutisch verträgliches Salz davon in Verbindung mit einem pharmazeutisch verträglichen Träger.

8. Eine Verbindung der Formel I oder deren pharmazeutisch verträgliches Salz zur Verwendung als Arzneimittel.

**Revendications**

1. Procédé pour la préparation de composés de formule I

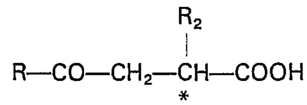 (I)

dans laquelle R représente un reste acide α-aminé correspondant à une molécule d'acide α-aminé dont on a séparé le groupe α-carboxyle, $R^2$ représente un atome d'hydrogène, un groupe alkyle ayant de 1 à 6 atomes de carbone, un groupe phényle ou phénylalkyle dont la partie phényle dans les deux groupes est non-substituée ou substituée par un groupe hydroxy, nitro ou méthoxy, et * indique que la configuration de l'atome de carbone marqué d'une astérisque est du type R ou S ou des types R et S combinés, procédé caractérisé en ce qu'il consiste à condenser un dérivé d'un acide α-aminé de formule II

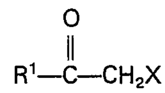 (II)

dans laquelle $R^1$ a la même signification que R ci-dessus, à condition que, si le reste acide α-aminé contient un groupe fonctionnel en plus du groupe α-aminé, le groupe α-amino et le groupe fonctionnel sont tous les deux protégés avec des groupes protecteurs usuels, et X représente le brome ou l'iode, avec un sel de métal alcalin d'un diester d'un acide malonique substitué ou non-substitué, de formule III

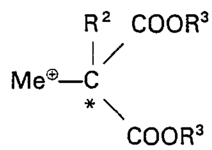 (III)

dans laqelle $R^2$ est tel que défini ci-dessus; $R^3$ représente un groupe carboxyl-protecteur et Me représente un métal alcalin; et * a la même signification que ci-dessus, de manière á former un compose de formule

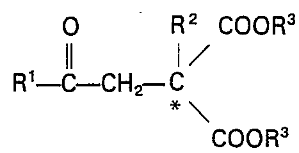

dans laquelle $R^1$, $R^2$ et $R^3$ sont tels que définis ci-dessus, puis à séparer l'un des groupes —$COOR^3$ par une réaction de décarboxylation connue dans la technique et finalement à éliminer les groupes protecteurs par des procédés connus dans la technique.

2. Procédé selon la revendication 1, caractérisé en ce que $R^3$ est un groupe alkyle en $C_1$—$C_4$ ou tétra-hydropyrannyle.

3. Procédé selon la revendication 1, caractérisé en ce que $R^2$ est l'hydrogène, un groupe alkyle ayant de 1 à 6 atomes de carbone, phényle ou benzyle, ces deux derniers groupes pouvant être non-substitués ou substitués dans le noyau par un groupe hydroxy, nitro ou méthoxy, et R est un groupe de formules:

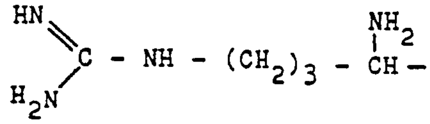

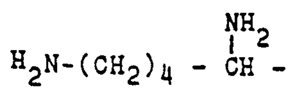

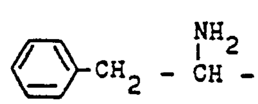

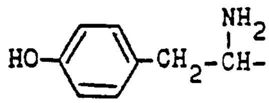

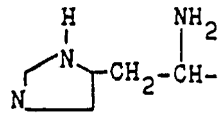

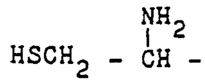

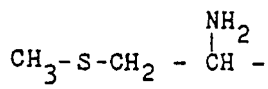

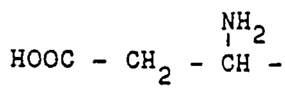

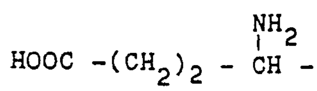

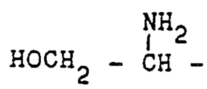

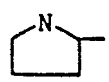

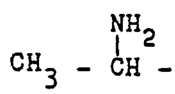

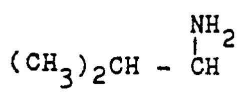

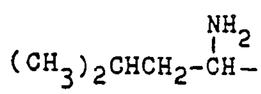

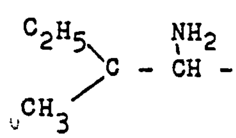 ou

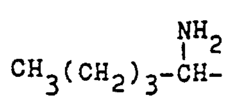

4. Procédé selon l'une quelconque des revendications 1 à 3, caractérisé en ce que la réaction de condensation est effectuée dans un solvant et à une température de 0 à 15°C.

5. Composé de formule I

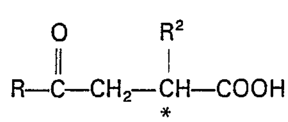

dans laquelle R, $R^2$ et * ont les significations indiquées dans la revendication 1, à condition que R ne soit pas un reste arginine correspondant à une molécule d'arginine dont le groupe α-carboxyle a été séparé si $R^2$ est le groupe benzyle ou un groupe p-hydroxybenzyle, et ses sels pharmaceutiquement acceptables.

6. Composé de formule I

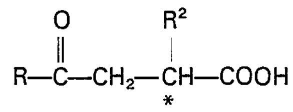

(I)

dans laquelle R, $R^2$ et * ont les significations indiquées dans la revendication 3, à condition que R ne soit pas un groupe de formule

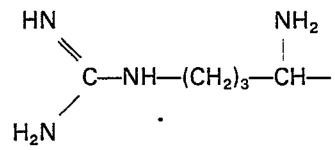

si $R^2$ est le groupe benzyle ou un groupe p-hydroxybenzyle, et ses sels pharmaceutiquement acceptables.

7. Composition pharmaceutique comprenant comme ingrédient actif un composé de formule I selon l'une des revendications 5 et 6 ou un de ses sels physiologiquement acceptables, associé avec un véhicule pharmaceutiquement acceptable.

8. Composé de formule I ou un de ses sels physiologiquement acceptables, utilisable comme médicament.